# EUROPEAN PATENT SPECIFICATION

(11) **EP 4 574 111 B1**
(45) Date of publication and mention of the grant of the patent: **20.05.2026**
(21) Application number: 24220103.6
(22) Date of filing: 16.12.2024
(51) Int. Cl.: A61F 9/008

(54) **OPHTHALMOLOGICAL DEVICE FOR SURGICAL TREATMENT OF A CORNEA OF AN EYE AND COMPUTER PROGRAM PRODUCT FOR CONTROLLING A PROCESSOR OF THE OPHTHALMOLOGICAL DEVICE**
OPHTHALMOLOGISCHE VORRICHTUNG ZUR CHIRURGISCHEN BEHANDLUNG EINER HORNHAUT EINES AUGES UND COMPUTERPROGRAMMPRODUKT ZUR STEUERUNG EINES PROZESSORS DER OPHTHALMOLOGISCHEN VORRICHTUNG
DISPOSITIF OPHTALMOLOGIQUE POUR LE TRAITEMENT CHIRURGICAL D'UNE CORNÉE D'UN IL ET PRODUIT PROGRAMME INFORMATIQUE POUR COMMANDER UN PROCESSEUR DU DISPOSITIF OPHTALMOLOGIQUE

(30) Priority: 21.12.2023 CH 14532023
(43) Date of publication of application: 25.06.2025
(60) Divisional application: 26168220.7
(73) Proprietor: Ziemer Ophthalmic Systems AG, 2562 Port (CH)
(72) Inventor: RATHJEN, Christian, 28197 Bremen (DE); STEINLECHNER, Michael, 8006 Zürich (CH)
(74) Representative: Rentsch Partner AG

(56) References cited:
- WO-A1-2016/209312
- US-A1- 2022 168 148
- US-A1- 2024 009 033
- US-A1- 2024 197 532

## Description

### FIELD OF THE DISCLOSURE

The present disclosure relates to an ophthalmological device for surgical treatment of a cornea of an eye of a patient and to a computer program product comprising a non-transitory computer-readable medium having stored thereon computer program code for controlling a processor of the ophthalmological device for surgical treatment of the cornea of the eye of the patient. The present disclosure relates in particular to an ophthalmological device comprising a laser source, focusing optics, a scanner system and an electronic circuit, which is configured to control the ophthalmological device.

### BACKGROUND OF THE DISCLOSURE

For the purposes of working on eye tissue by means of a laser beam, a work region is scanned by laser pulses by virtue of the pulsed laser beam being deflected in one or more scan directions by means of suitable scanner systems and converged onto a focal spot by a focusing optical module. In general, movable mirrors and / or moveable lens systems are conventionally used to deflect the light beams and/or the laser pulses, for example femtosecond laser pulses, said movable mirrors being pivotable about one or two scan axes, for example by way of galvano scanners, piezo scanners, polygon scanners, or resonance scanners. Further scan components, such as divergence modulators or z-modulators, are known for positioning and moving the focal spot in the eye tissue with respect to further scan axes.

US patent US 7,621,637 B2 describes exemplarily an apparatus for working on eye tissue, said apparatus having a base station with a laser source for producing laser pulses and a scanner, arranged in the base station, with movable deflection mirrors for deflecting the laser pulses in a scan direction. The deflected laser pulses are transferred via an optical relay system from the base station to an application head, the latter passing over a work region according to a scan pattern by means of a mechanically moved projection optical unit. According to US 7,621,637, in the application head, the deflection in the scan direction, which is much faster in comparison with the mechanical movement, is overlaid onto the mechanical movement of the projection optical unit and consequently onto the scan pattern thereof. A fast scanner system in the base station facilitates a fine movement of the laser pulses (micro-scan), which is overlaid on the scan pattern of the (mechanically) movable projection optical unit that covers a large work region, for example the entire eye.

US patent US 2024/009033 A1 describes exemplarily an ophthalmological apparatus comprising a laser source that is configured to produce a pulsed laser beam, a focusing optical unit that is configured to focus the pulsed laser beam into the eye tissue, and a scanner system for deflecting the pulsed laser beam onto work target points in the eye tissue.

Further, US patent US 2024/197532 A1 describes a method for forming a corneal flap in a patient's eye. A pocket cut is formed first below bed level, followed by the bed connected to the pocket cut, then by a side cut extending from the bed to the anterior corneal surface.

US patent US 2022/168148 A1 relates to an ophthalmic surgical laser system, comprising a laser delivery system for delivering a pulsed laser beam to a target in a subject's eye, an XY-scan device to deflect the pulsed laser beam, a Z-scan device to modify a depth of a focus of the pulsed laser beam, and a controller configured to form a top lenticular incision and a bottom lenticular incision of a lens in the subject's eye.

WO 2016/209312 A1 describes exemplarily a system for performing laser ophthalmic surgery is disclosed, including a mode-locked fiber oscillator-based ultra-short pulsed laser.

For processing or creating a pocket in the eye tissue, ophthalmological devices are known, such as scalpels devices or laser systems. The conventional available scanner systems enable to create a pocket within the cornea e.g. for removing a lenticule or for inserting a lens or for inserting cornea material. Further, a pocket within the cornea might be used for inserting pigment material, which might amend the visual appearance of an iris color of the eye. The known cutting patterns are not well suited for forming pockets as the known cutting patterns lack the desired precision and individuality required for up to date applications. In addition, the cornea of an eye may already comprise pigment material, which has been inserted in the cornea by surgery. It is in particular challenging to create a pocket within the cornea for removing such pigment material if required or if desired. Using conventional ophthalmological devices for the above-mentioned procedures might result in dangerous and harmful consequences for a patient's eye.

### SUMMARY OF THE DISCLOSURE

It is an object of the present disclosure to provide an ophthalmological device for surgical treatment of a cornea of an eye of a patient, which addresses at least some of the disadvantages of the known ophthalmological devices. In particular, it is an object of the present disclosure to provide an alternative and / or an improved ophthalmological device, which is in particular configured for cutting or forming at least one pocket inside the cornea of the eye of the patient.

According to the present disclosure, these objects are addressed by the features of the independent claims. Moreover, further advantageous embodiments emerge from the dependent claims and the description.

An ophthalmological device for surgical treatment of a cornea of an eye of a patient is specified. The ophthalmological device comprises preferably a laser source, focusing optics, a scanner system and an electronic circuit.

The laser source is in particular configured to generate a pulsed laser beam. The focusing optics is configured to make the pulsed laser beam converge onto a focal spot in the cornea of the eye of the patient and the scanner system is preferably configured to deflect the pulsed laser beam to target locations in the cornea. The scanner system may comprise only one scanner assembly arranged in one housing, which provides the required functionalities. In another embodiment, the scanner system may comprise a plurality of scanner assemblies arranged in one or a plurality of housings, which provide in combination with each other the required functionality. The ophthalmological device further comprises the electronic circuit, which is configured to control a first scanner device of the scanner system or of the scanner assembly to move the focal spot with a first scanning speed along a line, thereby forming a scan line. The electronic circuit is further configured to control a second scanner device of the scanner system or of the scanner assembly to move the scan line with a second scanning speed, comparatively slower than the first scanning speed, along a predetermined processing path, which extends inside the cornea from a peripheral portion of the cornea towards a center of the cornea or from a center portion of the cornea towards a peripheral portion of the cornea (vice versa), for cutting or forming a pocket inside the cornea. The first scanner device and the second scanner device might be implemented as separate scanner devices. In another embodiment, the first scanner device and the second scanner device are implemented as a single scanner device providing both functionalities. The electronic circuit is further configured to control the ophthalmological device to vary or modulate a geometrical parameter of the scan line and/or a physical parameter of the scan line, along the predetermined processing path through the cornea. The geometrical parameters might be described as qualities or characteristics that belong to geometric extensions, forms or shapes of the scan line. The physical parameters of the scan line might be described as any measurable property of the pulsed laser beam.

In an embodiment, the predetermined processing path is straight, in particular when viewed along the z-direction of the eye towards the eye. In another embodiment, the processing path is curved or bent.

The eye of the patient is a three dimensional object, which extends in three space direction, in particular in the x-, y- and the z-direction. The z direction is e.g. parallel with respect to a symmetry axis of the eye, parallel with respect to the visual axis of the eye or parallel with respect to a virtual axis crossing the center of the pupil and the apex of the cornea. Further, the z-direction might be perpendicular arranged to a plane of the eye, in particular the pupil plane or iris plate of the eye. The x-y directions might be arranged such that a virtual plane, which is determined by the x and y direction is arranged coplanar or parallel with the iris or pupil plane. Other definitions might also be conceivable.

In an embodiment, the geometrical parameters of the scan line comprise a length of the scan line, an orientation angle of the scan line with respect to the predetermined processing path, a curvature of the scan line, a position of the scan line with respect to the predetermined processing path and/or a depth of the focal spot in the cornea. The geometrical parameters might further include hiding or masking portions of the scan line along the predetermined processing path or hiding or masking the entire scan line along at least some portions of the predetermined processing path, such that no treatment is performed in the hidden portion.

In an embodiment, the processing path is curved or bent and/ or the shape of the scan line might vary along the curved or bent processing path.

In an embodiment, the physical parameters of the scan line comprise a pulse energy amount per pulse of the pulsed laser beam, or the pulse interval time between pulses of the pulsed laser beam. The physical parameters might further comprise a pulse-width, a pulse repetition rate and / or an average power. Additional physical parameters are also conceivable.

In an embodiment, the electronic circuit is configured to control the first scanner device or a specific geometrical parameter-determining device of the ophthalmological device to vary at least one of the geometrical parameters of the scan line, in particular to vary the length of the scan line along the predetermined processing path through the cornea. The specific geometrical parameter-determining device might be an adjustable aperture, a diaphragm and / or a screen. In order to vary at least one geometrical parameters, the electronic circuit controls the first scanner device and / or the specific geometrical parameter-determining device accordingly. E.g. an adjustable diaphragm arranged in the beam path might control the shape, in particular the length of the scan line. The length of the scan line lies e.g. in a range of 0,01 mm to 2 mm, preferably in a range of 0,05 mm to 1 mm. Further, the scan line might comprise a curvature as mentioned above, which might be modulated on the scan line by the first scanner device or a respective modulation device of the scanner system.

In a further embodiment, the electronic circuit is configured to control the first scanner device or a modulation device of the ophthalmological device to vary the curvature of the scan line along the processing path.

In an embodiment, the electronic circuit is further configured to control the second scanner device to move the scan line along the predetermined processing path, which extends from a first peripheral point of the cornea towards a diametrically opposed arranged second peripheral point of the cornea thereby crossing a center of the cornea, in particular a center area or center point. The cornea extend from the eye symmetry axis or the apex of the cornea towards the circumferentially arranged limbus and sclera. The center area of the cornea is for example a portion of the cornea extending from the center point towards the limbus. The center point of the cornea might be determined by the apex of the cornea, the intersection of the symmetry, visual or optical axis of the eye with the outer surface of the cornea or the intersection of a virtual axis with the outer surface of the cornea, the virtual axis being arrange perpendicular to the iris or pupil plane and crossing the iris or pupil venter. In another embodiment, the center area might extend along the cornea to a virtual extension of the pupil. The peripheral portion might be formed by the peripheral portion of the cornea and / or by the limbus and / or by a portion of the sclera, which borders the center area. The center area is in its smallest extension the center point of the cornea, through which the eye symmetry axis or the above described other eye axis may extend. In other words, the predetermined processing path, when viewed along the eye symmetry axis towards the eye (z-axis), extends preferably straight from one peripheral point in the cornea to a second point peripheral point, thereby crossing the center area, in particular the center point. It is to be noted that the predetermined processing path is a virtual path or line, which is virtually arranged in the cornea of the eye of the patient, and which determined the treatment path or treatment trajectory along which the scan line is moved.

In an embodiment, the electronic circuit is further configured to control the second scanner device to move the scan line along the predetermined processing path, which extends from a first point of the cornea towards a second point of the cornea thereby reaching or crossing the center of the cornea, in particular the center area or center point. The second point might be arranged in the center area.

In an embodiment, the electronic circuit is configured to control the scanner system, in particular the laser source or a laser interrupting device arranged in the beam path, to temporarily or partially interrupt the treatment of the cornea by the pulsed laser beam along the predetermined processing path, preferably when crossing the center area of the cornea. In other words, the pulsed laser beam is interrupted when the scan line reaches a first predetermined point along the predetermined processing path and might be switched on again, when reaching a second predetermined point. It is thereby possible to spare a portion, e.g. the center portion, from being treated by the pulsed laser beam. The processing path might be subdivided into its total length, which extends from the starting point to the end point, its effective length, which extends where the pulsed laser beam treats the cornea, and its ineffective length, which extends where the pulsed laser beam is interrupted e.g. switched off.

According to the invention, the electronic circuit is further configured to control the ophthalmological device to vary the geometrical parameter of the scan line, in particular the length of the scan line and / or the scan line orientation angle, along the predetermined processing path such that the length of the scan line shortens, preferably continuously shortens, towards the center of the cornea, and / or extends, preferably continuously extends, in the direction away from the center of the cornea, thereby forming at least partially a triangular shaped or a trapezoidal shaped pocket. Varying the at least one geometrical parameter of the scan line may comprise to vary the length of the scan line along the predetermined processing path and / or may comprise to vary the scan line orientation angle with respect to the predetermined processing path. E.g. reducing the length of the scan line along the processing path towards the center of the cornea starting from a peripheral portion of the cornea forms the desired triangular shaped or trapezoidal shaped pocket. Similarly, increasing the length of the scan line along the processing path from a center portion of the cornea towards a peripheral portion of the cornea forms the desired triangular shaped or trapezoidal shaped pocket. In addition, also varying the can line orientation angle, e.g. starting perpendicular to the processing path at the peripheral portion of the cornea to inclined to the processing path at the center portion of the cornea forms the desired at least partially triangular shaped or trapezoidal shaped pocket. The scan line length is preferably continuously shortened or continuously increased, such that the resulting pocket has a uniform outer contour, in particular without steps or changing lateral contour angles. In other words, the resulting lateral outer contour of the pockets follows a straight line or a continuous curve. By varying the scan line length and/or the orientation angle with respect to the predetermined processing path, it is possible to form the desired triangular shaped or trapezoidal shaped pocket.

In an embodiment, the electronic circuit is further configured to control the scanner system such that the scan line orientation angle with respect to the predetermined processing path remains unchanged or is kept constant during movement of the scan line along the predetermined processing path, in particular the scan line orientation angle remains or is kept at 90° with respect to the predetermined processing path. According to this embodiment, other geometrical parameters or physical parameters vary along the processing path.

According to the invention, the electronic circuit is further configured to control the scanner system to move the scan line along a plurality of predetermined processing paths, which are arranged at least partially next to each other, thereby forming a plurality of resulting pockets in the cornea. The electronic circuit is preferably configured to move the scan line along the plurality of predetermined processing paths successively. The plurality of processing paths are e.g. virtually arranged in the cornea are processed successively. Next to each other in this context may be defined as such that the arrangement and / or orientation of the resulting pockets in the cornea very, when the other parameters are kept unchanged. In other words, the resulting pockets do not overlap completely. The plurality of processing paths are arranged e.g. parallel next to each other or at an angle with respect to each other, in particular when viewed along the z-direction. The processing paths might cross each other or overlap partially. The processing paths in particular cross each other in the center area of the cornea.

In an embodiment, the electronic circuit is further configured to control the scanner system to move the scan line along the plurality of predetermined processing paths, wherein the electronic circuit is further configured to control the ophthalmological device to vary at least one of the physical parameters of the scan line along the predetermined processing paths through the cornea, and / or wherein the electronic circuit is further configured to control the ophthalmological device to vary at least one of the geometrical parameters of the scan line along the predetermined processing paths through the cornea.

According to a further embodiment, the electronic circuit is further configured to control the scanner system to move the scan line along the plurality of predetermined processing paths, wherein the electronic circuit is further configured to control the ophthalmological device to vary at least one of the physical parameters of the scan line along the predetermined processing paths through the cornea, wherein the physical parameters of the scan line along at least two predetermined processing paths differ from each other. For example, the pulse energy amount per pulse of the pulsed laser beam or the pulse interval time between pulses of the pulsed laser beam may vary along one predetermined processing path and between at least two predetermined processing paths. Furthermore, the electronic circuit is further configured to control the scanner system to move the scan line along the plurality of predetermined processing paths, wherein the electronic circuit is further configured to control the ophthalmological device to vary at least one of the geometrical parameters of the scan line along the predetermined processing paths through the cornea, wherein the physical parameters of the scan line along at least two predetermined processing paths differ from each other. It is thereby possible to create resulting pockets having different geometrical extensions as desired or required due to the anatomy of the eye of the patient.

In an embodiment, the electronic circuit is further configured to control the ophthalmological device, in particular the second scanner device, to move the scan line along the plurality of predetermined processing paths having varying length and / or having a different orientation with respect to the eye such that the plurality of resulting pockets have different geometrical extensions. According to this embodiment, at least two of the resulting pockets have different geometrical extensions with respect to each other. Varying or modulating at least one of the geometrical parameters of the scan line along the plurality of processing paths or having different processing paths with different (effective) lengths results in pockets having different geometrical extensions and shapes. Each eye, and in particular each cornea, is different, which might require different treatment, which is made available at least according to this embodiment.

In an embodiment, the electronic circuit is further configured to control the ophthalmological device, in particular the second scanner device, to move the scan line along the plurality of predetermined processing paths, which are arranged such that the plurality of resulting pockets are at least partially connected with each other, and / or wherein the electronic circuit is further configured to control the ophthalmological device to vary one of the geometrical parameters of the scan line such that the plurality of resulting pockets are at least partially connected with each other, thereby forming a connected pocket. According to this embodiment, at least two of the resulting pockets are partially connected with each other. This is realizable by respectively positioning the plurality of processing paths or by respectively controlling the geometrical parameters of the scan lines of the different processing paths. In other words, two resulting pockets of two scan lines partially overlap or are partially immediately bordering each other.

In an embodiment, the electronic circuit is further configured to control the ophthalmological device, in particular the second scanner device, to move the scan line along the plurality of predetermined processing paths, which are arranged around the center of the cornea, such that the connected pocket has at least partially a circular shape. Arranging a plurality of processing paths around the center area of the cornea enables to form a resulting pockets or a resulting connected pocket, surrounds or covers larger portions of the cornea, in particular the center area, or the entire cornea. The processing paths are preferably arranged regularly or symmetric around the center area. In another embodiment, the processing paths are preferably arranged irregularly around the center area, such that the resulting pockets are arranged irregularly. Further, the geometrical parameters of the scan line may vary across different processing paths, which creates further irregularities in the different resulting pockets.

In an embodiment, the electronic circuit is further configured to control the ophthalmological device to move the scan line along the plurality of predetermined processing paths, which are arranged around the center of the cornea and wherein the processing paths end prior to reaching the center area of the cornea such that the resulting connected pocket has at least partially a circular ring shape. The center area or at least portions of the center area are spared from being treated, such that the tip of the resulting pocket ends prior to reaching the center area or in particular the center point.

In an embodiment, the electronic circuit is further configured to control the scanner system and / or the focusing optics to vary a depth position of the focal spot within the cornea along the predetermined processing path. The depth position of the focal spot determines the position in z-direction of the scan line, and thereby the position in z-direction of the resulting pocket, within the cornea. By varying the z- position of the scan line within the cornea, it is e.g. possible to form respective pockets and a respective connected pocket with varying extension in z-direction. For example, it is possible that the pocket follows an outer or inner contour of the cornea with a predetermined distance (offset) to the outer or inner surface of the cornea. This distance might also change along the processing path. It is possible to individually create the resulting pockets based on the individual shape, in particular the individual outer or inner contour of the cornea, e.g. resulting from astigmatism.

In a further embodiment, the electronic circuit is further configured to control the scanner system and / or the focusing optics to vary a depth position of the focal spot along the line, which forms the scan line. This creates the possibility to further individually adapt the resulting pockets, or resulting connected pocket. In particular, it is possible to align the resulting pockets at least partially on the nearly spherical shape of the cornea.

In a further embodiment, the electronic circuit is configured to control the second scanner device of the scanner system to move the scan line multiple times along a single one of the predetermined processing paths. Further, the focusing optics is preferably configured to vary the depth of the focal spot, when the scan line is moved multiple times along the single one of the predetermined processing paths. In other words, the processing path seems unchanged when viewed along the eye axis, but the z-position of the scan line along the processing path, determined by the depth position of the focal spot, is changed. It is thereby possible to create a pocket with a larger extension or thickness, preferably predetermined thickness, in z-direction.

In a further embodiment, the electronic circuit is configured to control the first scanner device and/or the second scanner device to create a microstructure of the resulting pocket and / or of the resulting connected pocket, wherein the microstructure comprises irregularities, in particular the surfaces of the pockets comprise depressions, protrusions, or the edges are jagged or serrated. The microstructure of the resulting pocket is in particular the structure of their surfaces and their edges. The surfaces are e.g. smooth or comprise irregularities like depressions or protrusions. The irregularities in particular increase the adherence properties in case an eye colorant is inserted in the pocket or connected pocket.

In a further embodiment, the electronic circuit is further configured to control the second scanner device of the scanner system to move the scan line along multiple predetermined processing paths such that the resulting pockets, in particular the resulting connected pocket, envelops at least partially a lenticular tissue piece of the cornea. In other words, the electronic circuit controls the scanner device to cut the lenticular tissue piece when following the predetermined processing paths, which are respectively arranged.

In an embodiment, the electronic circuit is further configured to control the ophthalmological device to move the scan line along the at least one processing path, which is arranged in the cornea having a predetermined, preferably constant, distance or offset from the outer surface of the cornea, thereby following the outer contour of the cornea towards the center of the cornea. In other words, the z-position or depth position of the predetermined processing path, preferably determined by the depth of the focal spot, is arranged such that the processing path follows at least partially the outer contour of the cornea. The outer or exterior contour of the cornea is e.g. measured and the processing paths are arranged in dependence of the measurements, e.g. for cutting the desired lenticular tissue piece. In another embodiment, the inner contour or inner surface of the cornea or a virtual surface may be the reference surface.

In an embodiment, the ophthalmological device further comprises a patient interface comprising a contact body and one or more suction elements configured to fix the contact body to the cornea for contacting the cornea in a contacting zone where the contact body is in contact, preferably in applanating contact, with the outer surface of the cornea, and wherein the electronic circuit is further configured to control the scanner system to move the scan line along the predetermined processing path at least partially inside the contact zone. The patient interface is configured to advantageously position the eye of the patient with respect to the ophthalmological device, which improves the treatment of the cornea. The patient interface creates relatively rigid connection between the cornea and the ophthalmological device, in particular an application head of the ophthalmological device. Further, the contact body provides a defined, preferably planar, surface, on which the outer surface of the cornea adapts, when contacted. It is thereby possible to advantageously control the position of the resulting pockets within the cornea.

In an embodiment, the electronic circuit is further configured to control the scanner system to move the scan line along at least one predetermined access path, which extends from the outer surface of the cornea into the cornea, thereby connecting the surrounding with the at least one pocket enabling access to the pocket. According to this embodiment, an access is cut into the cornea, e.g. for inserting something (e.g. pigment material or a corneal inlay) into at least one of the resulting pocket or to remove something from the inside of the cornea, e.g. the lenticular tissue piece. The access might further be used for ventilation of the pockets, resulting gas might vent through the access out of the cornea. Further, the access or the plurality of accesses are used for removing the lenticular tissue piece or for inserting an implant (artificial or made from cornea material). In another embodiment, the access might be used for inserting pigment material into the resulting pocket, in particular for coloring the eye of the patient.

In an embodiment, the electronic circuit is further configured to control the scanner system, in particular to arrange the predetermined processing paths, to create the scan line and / or to vary the geometrical and/or physical parameters of the scan line, along the at least one predetermined processing path, based on patient individual data form the individual cornea of the patient. The patient individual data is preferably previously measured by respective ophthalmic devices. The patient individual data might include for example outer surface contour data of the cornea or thickness data of the cornea. The patient individual data is used, preferably in combination with treatment data, which determine the desired treatment or treatment result, for determining the position of the at least one processing path or the geometrical or physical parameters of the scan line along the at least one processing path.

According to a further aspect of the present disclosure, the ophthalmological device for surgical treatment, in particular for keratopigmentation is specified. The ophthalmological device comprises the laser source, the focusing optics, the scanner system and the electronic circuit as described above and hereinafter. The at least one resulting pocket is preferably used for housing pigment material for at least coloring the respective portion of the cornea. The electronic circuit is preferably configured to control the ophthalmological device, in particular the scanner device, to cut the desired pocket, plurality of pockets or connected pocket as desired by the patient for inserting the pigment or coloring material as described above or hereinafter. Every features described above or hereinafter might also form part of the further aspect of the present disclosure, in particular directed to an ophthalmological device for creating a pocket for keratopigmentation. The further aspect with respect to keratopigmentation, which might comprise the features described above or hereinafter could be made subject to at least one divisional application.

According to a further aspect, a computer program product comprising a non-transitory computer-readable medium having stored thereon computer program code is specified. The computer program code is used for controlling a processor of an ophthalmological device, which comprises a laser source configured to generate a pulsed laser beam, a focusing optics configured to make the pulsed laser beam converge onto a focal spot in the cornea, a scanner system configured to deflect the pulsed laser beam to target locations in the cornea. Whereby the computer program code is configured to control the processor such that the processor directs a fist scanner device of the scanner system to move the focal spot with a first scanning speed to target locations along a line, thereby forming a scan line. The computer program code is further configured to control the processor such that the processor direct a second scanner device of the scanner system to move the scan line with a second scanning speed, comparatively slower than the first scanning speed, along a predetermined processing path, which extends inside the cornea from a peripheral portion of the cornea towards a center of the cornea or vice versa, for cutting a pocket inside the cornea, and directs the ophthalmological device to vary at least one of: a geometrical parameter of the scan line or a physical parameter of the scan line, along the predetermined processing path through the cornea.

### BRIEF DESCRIPTION OF THE DRAWINGS

The herein described disclosure will be more fully understood from the detailed description given herein below and the accompanying drawings, which should not be considered limiting to the invention described in the appended claims. The drawings in which:
- **Figure 1**: shows a block diagram that schematically illustrates an ophthalmological device for surgical treatment of a cornea of an eye of a patient according to a first embodiment;
- **Figure 2**: shows a perspective schematic view of the ophthalmological device according to a second embodiment and a patient;
- **Figure 3**: shows a schematic view of an eye of a patient indicating scan lines following predetermined processing paths for the ophthalmological device according to a first embodiment;
- **Figure 4**: shows a schematic view of an eye of a patient indicating scan lines following predetermined processing paths for the ophthalmological device according to a second embodiment;
- **Figure 5**: shows a schematic view of an eye of a patient indicating scan lines following predetermined processing paths for the ophthalmological device according to a third embodiment;
- **Figure 6**: shows a schematic view of an eye of a patient indicating scan lines following predetermined processing paths for the ophthalmological device according to a fourth embodiment;
- **Figure 7**: shows a schematic view of an eye of a patient indicating scan lines following predetermined processing paths for the ophthalmological device according to a fifth embodiment;
- **Figure 8**: shows a schematic view of an eye of a patient indicating scan lines following predetermined processing paths for the ophthalmological device according to a sixth embodiment;
- **Figure 9**: shows a cross section of an eye of a patient indicating scan lines following predetermined processing paths for the ophthalmological device according to a first embodiment;
- **Figure 10**: shows a cross section of an eye of a patient indicating scan lines following predetermined processing paths for the ophthalmological device according to a second embodiment;
- **Figure 11**: shows a cross section of an eye of a patient indicating scan lines following predetermined processing paths for the ophthalmological device according to a third embodiment;
- **Figure 12**: shows a cross section of an eye of a patient indicating scan lines following predetermined processing paths for the ophthalmological device according to a fourth embodiment;
- **Figure 13**: shows a cross section of an eye of a patient indicating scan lines following predetermined processing paths for the ophthalmological device according to a fifth and sixth embodiment.

### DESCRIPTION OF THE EMBODIMENTS

Reference will now be made in detail to certain embodiments, examples of which are illustrated in the accompanying drawings, in which some, but not all features are shown. Indeed, embodiments disclosed herein may be embodied in many different forms and should not be construed as limited to the embodiments set forth herein; rather, these embodiments are provided so that this disclosure will satisfy applicable legal requirements. Whenever possible, like reference numbers will be used to refer to like components or parts.

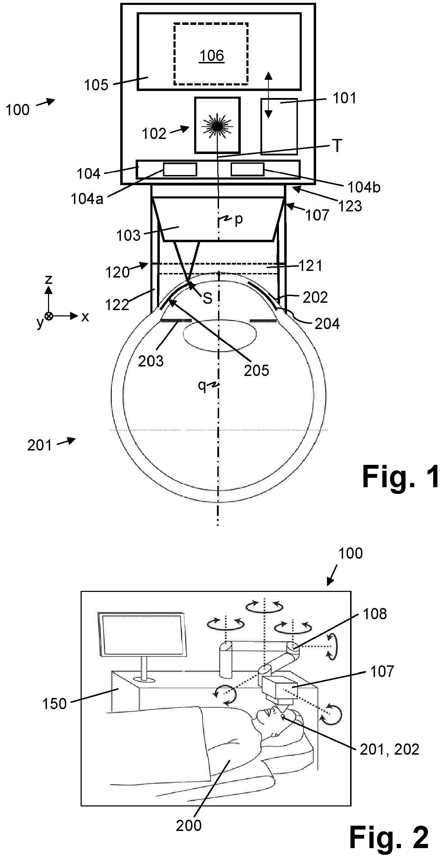

**Figure 1** schematically shows an ophthalmological device 100 for surgical treatment of a cornea 202 of an eye 201 of a patient 200. The ophthalmological device 100 uses a pulsed laser beam T for treating the cornea 202. Specifically, the ophthalmological device 100 is configured to treat the cornea 202 and adjacently arranged eye tissue e.g. limbus sclera, lens etc., by irradiating the respective eye issue or by cutting the respective eye tissue. The person skilled in the art will understand that by controlling the ophthalmological device 100, in particular its components, respectively, it is possible to cut three-dimensional tissue pieces, e.g. lenticular tissue pieces 205, which can be removed from the eye, e.g. a lenticular tissue piece 205 of the cornea 202 is removed from within the cornea 202 for refractive correction of the cornea 202. The person skilled in the art will further understand that by controlling the ophthalmological device 100, in particular its components, respectively, it might be further possible to achieve three-dimensional volume treatment of the eye tissue, e.g. for generating a pocket, a void volume inside the eye tissue e.g. the cornea 202, e.g. for refractive correction of the cornea 202 and/or for other purposes, such as for inserting implants, eye colorant or pigment material into the cornea 202.

As illustrated schematically in Figure 1, the ophthalmological device 100 comprises a laser source 102 for generating the pulsed laser beam T, a focusing optics 103 for focusing or making the pulsed laser beam T converge onto a focal spot S in or on the eye tissue, and a scanner system 104 for moving the focal spot S to target locations in and/or on the eye tissue, in particular configured to deflect the pulsed laser beam T to target locations in the cornea 202.

The ophthalmological device 100 further comprises an electronic circuit 105 for controlling the ophthalmological device 100, in particular the laser source 102 and the scanner system 104. The electronic circuit 105 implements a programmable control device and comprises e.g. one or more processors 106 with program and data memory and programmed software modules for controlling the processors 106, and/or other programmable circuits or logic units such as ASICs (application specific integrated circuits) or the likes. In an embodiment, a functional part of the electronic circuit 105 is arranged in a separate housing and implements a further programmable control device 105*, e.g. a computer system, comprising e.g. one or more processors 106* with program and data memory and programmed software modules for controlling the processors 106*, and/or other programmable circuits or logic units such as ASICs or the like. Depending on the embodiment and/or configuration, a communication link might be required, which comprises one or more electrical connections, an electronic bus, a wired communication network, and/or a wireless communication network.

The laser source 102 comprises e.g. a femtosecond laser for producing femtosecond laser pulses, which have pulse widths of typically 10 fs to 1000 fs (1 fs = 10 15 s). The laser source 102 is arranged in a separate housing or in a housing shared with the focusing optics 103.

The focusing optics 103 is configured to focus the pulsed laser beam T or the laser pulses, respectively, onto a focal spot S in or on the eye tissue, i.e. for making the pulsed laser beam T converge to a focus or focal spot in or on the eye tissue. The focusing optics or optical module 103 comprises one or more optical lenses. In an embodiment, the focusing optics 103 comprises a focus adjustment device for setting the focal depth of the focal spot S, for example one or more movable lenses, in the focusing optics 103 or upstream of the focusing optics 103, or a drive for moving the entire optics 103 along the projection axis p (z-axis). By way of example, the focusing optics 103 is installed in an application head 107, which can be placed onto the eye 201. The person skilled in the art will understand that in cases where the focusing optics 103 is adjusted (focus) or moved as part of the scanning process or scanning actuation, the focusing optics 103 and associated drives (actuators) can be viewed and considered as parts of the scanner system 104, implementing a z-scanner configured to modulate the depth z of the focal spot S along the projection axis p or the eye symmetry axis q.

As illustrated schematically in Figure 1, the ophthalmological device 100 comprises a patient interface 120 for attaching the application head 107 or the focusing optics 103, respectively, onto the eye 201. Depending on the embodiment, the patient interface 120 is connected to the application head 107 in a fixed or removable manner. The patient interface 120 comprises an optional contact body 121 and one or more suction elements configured to fix the contact body 121 and thus the patient interface 120 to the cornea 202. For example, the one or more suction elements are arranged in a fastening ring 122, e.g. a vacuum-controlled suction ring, whereby the one or more suction elements are connected fluidically to a suction pump. The contact body 121, also referred to as applanation body, is at least partly light-transparent. In the state where the patient interface 120 or the contact body 121, respectively, is fixed to the cornea 202, the cornea 202 is applanated where the contact body 121 is in contact with the exterior (anterior) surface of the cornea 202.

In an embodiment, the ophthalmological device 100 further comprises a measurement system 101 configured to determine positional reference data of the cornea 202. Depending on the embodiment, the measurement system 101 comprises a video capturing system, an optical coherence tomography (OCT) system, and/or a structured light illumination system. Accordingly, the measurement data or positional reference data determined by the measurement system 101 includes video data, including top view data (comprising two-dimensional images), and/or OCT data of the cornea 202 (comprising three-dimensional tomography data). The measurement system 101 is configured to determine the positional reference data of the cornea 202 also in an applanated state of the cornea 202. The measurement system 101 is connected to and/or integrated with the electronic circuit 105, which is further configured to control ophthalmological device 100, in particular the scanner system 104, using the data, in particular the positional reference date or further data, from the measurement system 101.

The measurement system 101, in particular the OCT integrated in the respective ophthalmological device, might further be configured to determine the position reference data of the cornea 202 during treatment of the cornea 202, in particular continuously, such that cutting of the pocket 170 may be continuously monitored. The determined position reference data during treatment may be continuously matched to expected data, in particular expected reference data. In case the determined position reference data during treatment deviate from the expected data, in particular when the deviation reaches or fulfills a predetermined threshold, the electronic circuit might adjust the control data for the scanner system 104 or the entire ophthalmological device 100, in particular in real time. In case a strong deviation e.g. surpassing the threshold by 25% or more is detected, the electronic circuit might be configured to interrupt the treatment.

Aside from the optional positional reference data from the measurement system 101, the electronic circuit 105 is configured to use treatment control data to control the ophthalmological device 100, in particular the scanner system 104 to move the focal spot S along the line forming a scan line 130, which is further moved in or on the eye tissue to target locations along a processing path 160 defined by the treatment control data for treating in and/or on the eye tissue. The treatment control data is e.g. stored in the data memory and/or data store of the electronic circuit 105 and/or received via a communication link from a separate computer system.

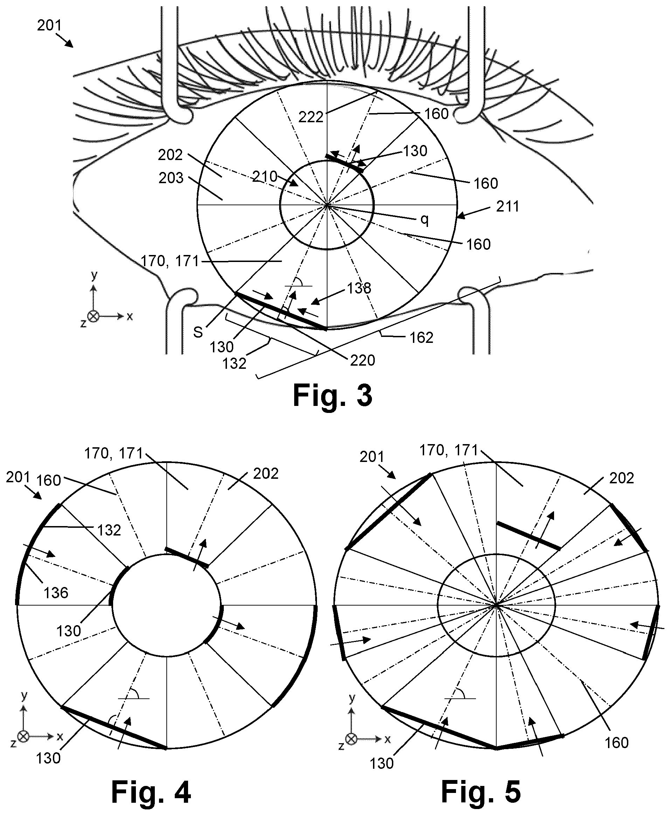

Essentially, the treatment control data defines the position and orientation of the at least one processing paths 160 along which the scanner system 104 is to move the scan line 130 the focal spot S for treating the eye tissue. Further, the orientation, arrangement, geometrical parameters and physical parameters of the scan line 130 along the predetermined processing paths 160 might also be determined by the treatment control data. In effect, the treatment control data defines a sequence of consecutive target locations in a three-dimensional x/y/z-space for treating e.g. a three-dimensional area in the cornea 202. Depending on the embodiment and/or configuration, for defining the at least one processing path 160, the treatment control data comprises path definition data, e.g. coordinates, positions, positional references, etc., and/or path processing data, e.g. instructions, operations, and/or procedures for the scanner system 104 and its components, described below in more detail. As illustrated schematically in Figures 3, 4, 5, 6, 7, 8, 9, 10, 11, 12 or 13, the at least one processing path 160 can be defined as a trajectory for the scan line 130, formed by the focal spots S, to be moved along by the scanner system 104 for treating the cornea 202. The treatment control data may further comprise control data for controlling the laser source 102 e.g. for controlling physical parameters of the pulsed laser beam T like processing speed, pulse rate, pulse energy, etc..

Treating the eye tissue, in particular the cornea 202 imposes scanning requirements that must be met by a scanner system 104. More specifically, the scanning requirements are determined by the dynamics involved in moving the focal spot S and the resulting scan line 130 along the processing path 160, defined by the treatment control data, for treating the eye tissue. The dynamics include the speed with which a focal spot is to be placed and moved along the scan line 130 and the processing path 160 and the distances that must be covered by the focal spot S within a given time, at a defined speed, with a defined acceleration, and/or with a defined speed of acceleration (jerk/jump). The scanning requirements include a depth-scanning requirement, representing the required modulation of the depth z of the focal spot S when moved along the processing path 160 defined in particular by the treatment control data. The depth-scanning requirement includes the dynamics required for the modulation of the depth z of the focal spot S, the scan line 130 respectively along the processing path 160 defined in particular by the treatment control data. The required depth scanning dynamics may comprise the required depth scanning speed, the required depth scanning frequency, the required amplitude of depth modulation at a particular speed of the depth modulation, the required amplitude of depth modulation at a particular frequency of the depth modulation, the required acceleration of the depth modulation, and/or the required speed of the acceleration of the depth modulation.

**Figure 2** illustrates in a perspective view the ophthalmological device 100, in particular illustrating some of its components and a patient 200, which is treated by the ophthalmological device 100. The ophthalmological device 100 comprises a base station 150, which is configured as a fixed or mobile apparatus. The base station 150 houses e.g. the laser source 102, at least partially the scanner system 104 and / or the electronic circuit 105. The base station 150 further might include, for example, a power supply and other auxiliary subsystems necessary for operation of the ophthalmological device 100. Figure 2 further shows a moveable arm 108, which comprises mirrors and which is configured to guide the pulsed laser beam T to the eye 201 of the person 200. The arm 108 extends from the base station 150 to the application head 107, which e.g. contacts via the patient interface 120 the eye tissue of the person 200.

**Figure 3** shows a schematic view of an eye 201 of a patient 200. Figure 3 further indicates a plurality of processing paths 160 along which the scan line 130 is moved for treating the cornea 202 of the patient 200 according to a first embodiment. Figure 3 shows the entire eye 201 of the patient 200 comprising the cornea 202, the pupil in the center and the surrounding iris 203. Further, the processing paths 160, which are according to this embodiment arranged uniformly around the center of the cornea 202. All of the processing paths 160 cross the center area 210 of the cornea 202 determined by the symmetry axis q of the eye 201. Figure 3 shows that the focal spot S is moved by the scanner system 104, in particular by the first scanner device 104a of the scanner system, along a line with a first scanning speed, such that the scan line 130 is formed. It is further visible and indicated by arrows that the scan line 130 is moved by the scanner system 104, in particular by the second scanner device 104b of the scanner system 104, with a second scanning speed comparatively slower than the first scanning speed, along the predetermined processing path 160, in particular along a plurality of predetermined processing paths 160, from a first peripheral point 220 of the cornea 202 towards a center area 210 of the cornea 202, crossing the center area 210 and again towards a second peripheral point 222 of the cornea 202, which is arranged diametrically opposed with respect to the first peripheral point 220. Figure 3 further illustrates that the length 132 of the scan line 130 shortens from the first peripheral point 220 towards the center 210 of the cornea 202 and expands again from the center 210 of the cornea 202 towards the second peripheral point 222. In other words, the ophthalmological device 100 is controlled by the electric circuit 105 to vary the length 132 of the scan line 130 as geometrical parameter of the scan line 130 when moved along the predetermined processing path 160 through the cornea 202. The respective movement of the scan line 130, which might be called as the surgical treatment, results in a pocket 170, which has the shape of the scanned area of the scan line 130. According to Figure 3, all of the resulting pockets 170 have the shape of triangles. The processing paths 160 and the geometrical parameters, in particular the scan line length 132 of the scan line 130 are determined such that the resulting pockets 170 are at least partially connected with each other thereby forming at least one connected pocket 171 made of at least two pockets 170. The connected pocket 171 has the shape of approximately a circle at least when viewed along the z-axis towards the eye 201. Figure 3 shows that the scan line 130 is arranged straight and both endpoints of the scan line 130 have the same distance from the processing path 160. Further, the scan line 130 is according to this embodiment arranged perpendicular which respect to the processing path 160. In other words, the processing path 160 is a symmetry axis for the scan line 130. A scan line position 138 with respect to the processing path 160 is kept constant along the processing path 160. The center point of the scan line 130 remains in line with the processing path 160 when the scan line 130 is moved along the processing path 160.

**Figure 4** shows a further schematic view of an eye 201 of a patient 200. Figure 4 further indicates a plurality of processing paths 160 along which the scan line 130 is moved for treating the cornea 202 of the patient 200 according to a second embodiment. The second embodiment deviates from the first embodiment in particular by the arrangement of the processing paths 160. The processing paths 160 start e.g. from a peripheral point 220 of the cornea 202 but do not cross the center 210 of the cornea 202, they end prior. The processing paths 160 end at a peripheral point 222 or the cornea 202 but do not start in the center point of the cornea 202, they start from a peripheral point. Further, the length 132 of scan line 130 does not shortens until it reaches a point shape or do not start having a point shape. The resulting pocket 170 have therefore a trapezoid shape. The processing paths 160 end or start in particular where the iris 203 of the eye 201 ends or starts. The resulting connected pocket 171 has at least partially a ring, torus or donuts like shape, covering at least partially the iris when viewed along the z-axis. Further, Figure 4 shows two embodiments of the scan line 130. According to the first embodiment, also shown in Figure 3, the scan line 130 is straight, in other words follows a straight line. According to the second embodiment, the scan line 130 has a scan line curvature 136, which follows in particular a circular arc. The radius of the scan line curvature 136 might remain constant or may shortens or widen when the scan line 130 is moved along the processing path 160, the latter is shown in Figure 4. The radius of the scan line curvature 136 might correspond to the iris radius. In addition, also the scan line length 132 shortens towards the center area. In other words, the electronic circuit 105 controls the ophthalmic device 100, in particular the first scanner device 104a to vary the curvature as geometrical properties along the predetermined processing path 160 and at the same time, the electronic circuit 105 controls the ophthalmic device 100, in particular the first scanner device 104a to vary the length 132 of the scan line 130 along the processing path 160. Further, the scan line 130 might additionally be curved three-dimensional, in particular such that it follows the shape of the cornea 202, the approximately spherical shape of the cornea 202.

**Figure 5** shows a further schematic view of an eye 201 of a patient 200. Figure 5 further indicates a plurality of processing paths 160 along which the scan line 130 is moved for treating the cornea 202 of the patient 200 according to a third embodiment. The second embodiment deviates from the former embodiments at least in that the plurality of the processing paths 160 have different lengths 162 thereby forming an oval or elliptical connected pocket 171. In addition, the different scan lines 130 have different scan line lengths 132. Nevertheless, the arrangement and orientation of the processing paths 160 and the length 132 of the scan lines 130 are adjusted such that the resulting pockets 170 are at least partially connected with each other. The scan lines 130 as shown in Figure 5 are straight, but it is of course also conceivable that the scan lines 130 would have a curvature 132. In addition, Figure 5 shows that the predetermined processing paths 160 extend from one peripheral point of the cornea 202 to another peripheral point of the cornea thereby crossing the center area of the cornea 202. Further, the scan line length 132 shortens towards the center area, in particular continuously shortens, and the scan line length 132 extends away from the center area, in particular continuously extends. In another embodiment, not shown, the processing path 160 might comprise a bend or a curvature, when viewed along the z-axis. The bend is for example arranged in the center area of the cornea 202.

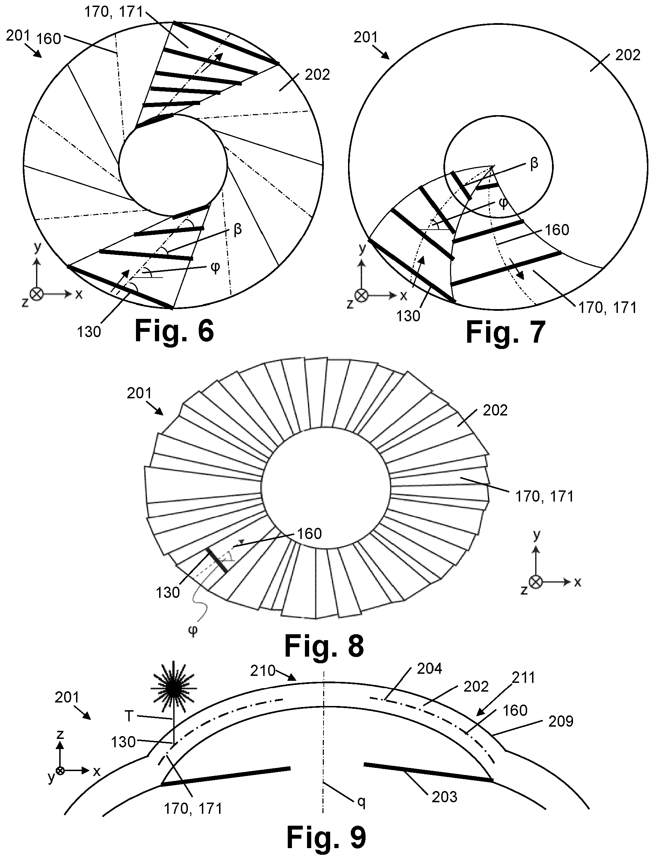

**Figure 6** shows a further schematic view of an eye 201 of a patient 200. Figure 6 further indicates a plurality of processing paths 160 along which the scan line 130 is moved for treating the cornea 202 of the patient 200 according to a fourth embodiment. Figure 6 shows that the processing path 160 extends from a peripheral point towards the center area 210 but does not reach or cross the center point. In addition, the processing path 160 ends at the expected iris end when viewed along the z-axis. The scan line length 132 varies along the processing paths 160. Further, also a scan line orientation angle β varies as geometrical parameter along the processing path 160.

**Figure 7** shows a further schematic view of an eye 201 of a patient 200. Figure 7 further indicates a plurality of processing paths 160 along which the scan line 130 is moved for treating the cornea 202 of the patient 200 according to a fifth embodiment. According to the fifth embodiment, the processing paths 160 comprise a curvature and extend from a peripheral point of the cornea 202 to the center area in particular the center point of the cornea 202. In other words, a processing path angle φ varies along the processing path. The scan line length 132 and the scan line orientation angle β vary as geometrical parameter along the processing path 160.

**Figure 8** shows a further schematic view of an eye 201 of a patient 200. Figure 8 further indicates a plurality of processing paths 160 along which the scan line 130 is moved for treating the cornea 202 of the patient 200 according to a sixth embodiment. The embodiment of Figure 8 shows a combination of the previous embodiment. In particular, Figure 8 shows many pockets 170 surrounding the center area of the cornea 202. In addition, Figure 8 shows that the different pockets 170 have varying geometrical extensions, which result from respective positioning of the processing paths 160 and the respective controlling of the geometrical parameters of the scan line 130. The pockets 170 form one connected pocket 171. Figure 8 advantageously shows the embodiment in which the different pockets 170 have different radial extensions, which results in a jagged or serrated outer radial contour and/or inner radial contour of the resulting connected pocket 171. It is visible that, the microstructure of the different pockets 170 is different across the different pocket 170. In particular, the surface extensions, the surface shape and the shape of the respective edges varies across the different pockets 170. These irregularities and the specific embodiment of the resulting connected pocket 171 provide the advantage that an eye colorant 204 when inserted may be distributed unevenly in the resulting connected pocket 171, which results in a realistic and/or individual visual appearance. Further, an adherence of pigments of the eye colorant 204 within the resulting connected pocket 171 is improved.

The Figures 3 to 8 focus on different embodiments of the resulting pockets 170, connected pocket 171, different embodiments of processing paths 160 and different embodiments of the scan line 130, which form the respective pockets 170, when viewed along the z-axis. In other words, the Figures 3 to 8 show possible embodiments with respect to the x und y axis as shown in the Figures, variations in the depth / in the z direction are not shown with respect to the Figures 3 to 8 but will be shown with respect to the following figures. All of the shown embodiments are of course combinable.

**Figure 9** shows a cross section of an eye 201 of a patient 200. The cross section indicates in particular a scan line 130 following at least one of the predetermined processing paths 160 according to a first embodiment. Figure 9 shows in particular the iris 203, the cornea 202, the sclera and the limbus of the eye 201 of the patient 200. Figure 9 shows that the processing paths 160 follow the contour of the cornea 202. In other words, the processing paths 160 have different depth positions or positions in z-direction from the peripheral portion of the cornea 202 towards the center area of the cornea 202. In particular, the depth of the focal spot S is controlled by the ophthalmological device 100, in particular by the focusing optics 103, such that the z-position of the focal spot varies (moves away from the eye center or moves towards the eye center) when the scan line 130 is moved towards the center area 210 of the cornea 202. It is thereby possible to move the scan line 130 in three dimensions within the cornea 202, which creates three-dimensional pockets 170. Figure 9 shows that the resulting connected pocket 171 might cover the iris 202 when viewed from above along the z-axis. The connected pocket 171 is for example used for inserting an eye colorant 204, which covers the natural color of the iris 202. The different pockets 170 might be used to house different eye colorants 204 with different pigments for creating the desired visual appearance. Further, the different pockets 170 might have different extensions; in particular also in z-direction such that the thickness of eye colorant 204 varies across the pockets 170 and also within one pocket 170 for creating the desired visual appearance. In another embodiment, the different pockets 170, which form together the connected pocket 171, or the scan lines 130, which form the respective pockets 170 are slightly tilted with respect to each other, thereby creating the desired microstructure of the connected pocket 171, in particular for an advantageous adhesion of the eye colorant 204. The tilt or the inclination of the different scan lines 130 with respect to each other or of the different pockets 170 might be implemented by a respective modulation of the focal spot S or by tilting the entire scan line e.g. by tilting a respective optical module.

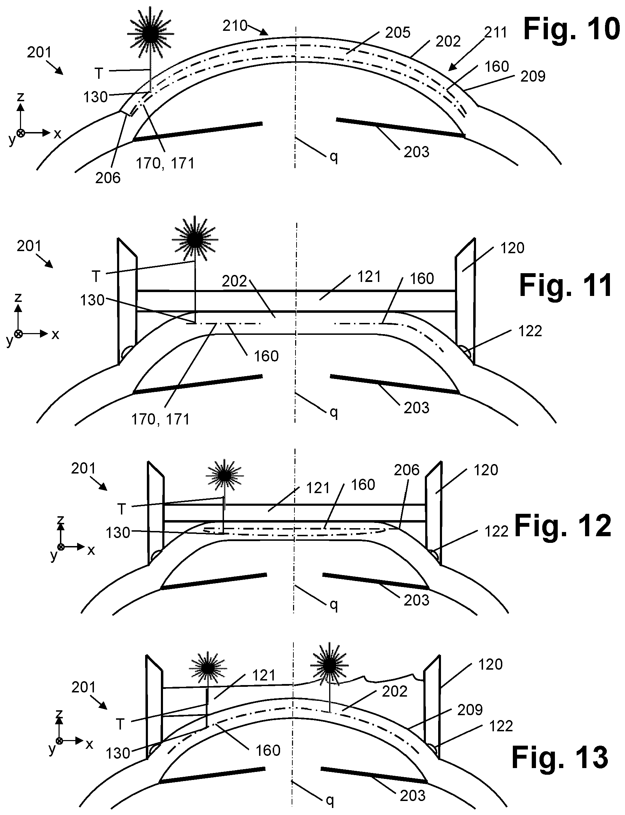

**Figure 10** shows a cross section of an eye 201 of a patient 200. The cross section indicates in particular a scan line 130 following at least one of the predetermined processing paths 160 according to a second embodiment. According to this embodiment, a plurality of processing paths 160 is arranged at least partially overlapping when viewed along the z-axis. In other words, the processing paths 160 are arranged at least partially at different focal depths and in particular start form the same starting points and end at the same end points such that the resulting pockets 170 or resulting connected pocket 171 envelops a lenticular tissue piece 205. In other words, the ophthalmological device 100 is used to cut by the pockets 170 the lenticular tissue piece 205 from the cornea 202. The lenticular tissue piece 205 might be removed through an access 206 or access channel from the cornea 202.

**Figure 11** shows a cross section of an eye 201 of a patient 200. The cross section indicates in particular a scan line 130 following at least one of the predetermined processing paths 160 according to a third embodiment. According to this embodiment, a patient interface 120 is arranged on the eye 201 of the patient. The patient interface 120 comprises a contact body 121 which contacts and applanates the cornea 202, in particular the outer surface 209 of the cornea 202. The patient interface 120 further comprises the fastening ring 122, which is configured to position the patient interface 120 on the eye 201. The fastening ring 122 might comprise the suction opening, which provides a vacuum for advantageous fixation. Figure 11 further shows two embodiments of processing paths 160 along which the scan line 130 might be moved. According to the first embodiment, shown left, the processing path 160 is arranged straight, with respect to the z-direction, the focal depth is constant. Further, the processing path 160 according to the first embodiment is completely arranged in the applanating zone of the cornea 202. According to the second embodiment, shown right, the processing path additionally extends beyond the applanating zone and follows the outer spherical shape of the cornea 202. In other words, the processing path 160 might be subdivided into a first straight portion and a second portion with varying focal depth.

**Figure 12** shows a cross section of an eye 201 of a patient 200. The cross section indicates in particular a scan line 130 following at least one of the predetermined processing paths 160 according to a fourth embodiment. According to this embodiment, the processing paths 160 and the respective scan lines 130 are arranged and configured such that a lenticular tissue piece 205 is formed by the resulting pockets 170 or cuts. The lenticular tissue piece 205 might be removed through the access 206. Further, a lens or another object might be inserted through the access 206 into the resulting pocket 170 or connected pocket.

**Figure 13** shows a cross section of an eye 201 of a patient 200. The cross section indicates in particular a scan line 130 following at least one of the predetermined processing paths 160 according to a fifth and sixth embodiment. The fifth embodiment is shown left and the sixth embodiment is shown right. The fifth embodiment deviates from the third and fourth embodiment at least in the contact body 121 of the patient interface 120. The contact body 121 of the fifth embodiment follows the natural shape of the cornea 202. In other words, the outer surface of the contact body 121, which is configured to contact he cornea 202 has e.g. a spherical shape, such that contact body 121 adapts advantageously to the outer surface 209 of the cornea 202. The sixth embodiment deviates from the third, fourth and sixth embodiment in that the patient interface 120 does not comprise a contact body. According to this embodiment a liquid solution is arranged in the patient interface in particular between the application head 107 and the outer surface 209 of the cornea 202. This embodiment of the patient interface might be called liquid interface.

### LIST OF REFERENCE SYMBOLS

- 100: ophthalmological device
- 101: measurement system / imaging means
- 102: laser source
- 103: focusing optics
- 104: scanner system
- 104a: first scanner device
- 104b: second scanner device
- 105: electronic circuit
- 106: processor
- 107: application head
- 108: arm
- 120: patient interface
- 121: contact body
- 122: fastening ring
- 123: scan line length determining device
- 130: scan line
- 132: scan line length
- 136: scan line curvature
- 138: scan line position
- 140: depth of the focal spot (S)
- 142: pulse energy amount
- 150: base station

- 160: processing path
- 162: length of the processing path
- 170: pocket
- 171: connected pocket

- 200: patient
- 201: eye
- 202: cornea
- 203: iris
- 204: eye colorant
- 205: lenticular tissue piece
- 206: access
- 209: outer surface of the cornea
- 210: center area of the cornea
- 211: peripheral area of the cornea
- 220: first peripheral point
- 222: second peripheral point

- S: Focal spot
- T: Pulsed laser beam
- φ: processing path angle
- β: scan line orientation angle
- p: projection axis
- q: symmetry axis

## Claims

1. An ophthalmological device (100) for surgical treatment of a cornea (202) of an eye (201) of a patient (200), the ophthalmological device (100) comprising:
a. a laser source (102) configured to generate a pulsed laser beam (T);
b. a focusing optics (103) configured to make the pulsed laser beam (T) converge onto a focal spot (S) in the cornea (202);
c. a scanner system (104) configured to deflect the pulsed laser beam (T) to target locations in the cornea (202); and
d. an electronic circuit (105) configured to:
i. control a first scanner device (104a) of the scanner system (104) to move the focal spot (S) with a first scanning speed along a line, thereby forming a scan line (130),
ii. control a second scanner device (104b) of the scanner system (104) to move the scan line (130) with a second scanning speed, comparatively slower than the first scanning speed, along a predetermined processing path (160), which extends inside the cornea (202) from a peripheral portion of the cornea (202) towards a center of the cornea (202) or vice versa, for treating the cornea (202), in particular for cutting a pocket (170) inside the cornea (202), and
iii. control the ophthalmological device (100) to vary at least one of: a geometrical parameter of the scan line (130) or a physical parameter of the scan line (130), along the predetermined processing path (160) through the cornea (202), and
iv. control the ophthalmological device (100) to vary the geometrical parameter of the scan line (130) along the predetermined processing path (160) such that a length (132) of the scan line (130) shortens in the direction towards the center of the cornea (202), and/or extends in the direction away from the center of the cornea (202), thereby forming at least partially a triangular shaped or a trapezoidal shaped pocket (170), and/or
v. control the scanner system (104) to move the scan line (130) along a plurality of predetermined processing paths (160), which are arranged at least partially next to each other, thereby forming a plurality of resulting pockets (170) in the cornea (202).

2. The ophthalmological device (100) according to claim 1, wherein the geometrical parameters of the scan line (130) comprise at least one of: the length (132) of the scan line (130), an orientation angle (β) of the scan line (130) with respect to the predetermined processing path (160), a curvature (136) of the scan line (130), a position (138) of the scan line (130) with respect to the predetermined processing path (160) or a depth (140) of the focal spot (S) in the cornea (202).

3. The ophthalmological device (100) according to any one of the preceding claims, wherein the physical parameters of the scan line (130) comprise at least one of: a pulse energy amount (142) per pulse of the pulsed laser beam (T) or pulse interval time between pulses of the pulsed laser beam (T).

4. The ophthalmological device (100) according to any one of the preceding claims, wherein the electronic circuit (105) is configured to control the first scanner device (104a) or a specific geometrical parameter determining device (123) of the ophthalmological device (100) to vary at least one of the geometrical parameters of the scan line (130), in particular to vary the length (132) of the scan line (130) along the predetermined processing path (160) through the cornea (202).

5. The ophthalmological device (100) according to any one of the preceding claims, wherein the electronic circuit (105) is further configured to control the second scanner device (104b) to move the scan line (130) along the predetermined processing path (160), which extends from a first peripheral point of the cornea (220) towards a diametrically opposed arranged second peripheral point of the cornea (222) thereby crossing the center, in particular the center point or center area (210), of the cornea (210).

6. The ophthalmological device (100) according to any one of the preceding claims, wherein the electronic circuit (105) is further configured to control the scanner system (104) such that the scan line orientation angle (β) with respect to the predetermined processing path (160) is kept constant during movement of the scan line (130) along the predetermined processing path (160), in particular the scan line orientation angle (β) remains at 90° with respect to the predetermined processing path (160).

7. The ophthalmological device (100) according to any one of the preceding claims, wherein the electronic circuit (105) is further configured to control the ophthalmological device (100), in particular the second scanner device (104b), to move the scan line (130) along the plurality of predetermined processing paths (160) having varying lengths (160), and / or having a different orientation with respect to the eye (201) such that the plurality of resulting pockets (170) have different geometrical extensions.

8. The ophthalmological device (100) according to any one of the preceding claims, wherein the electronic circuit (105) is further configured to control the ophthalmological device (100), in particular the second scanner device (104b), to move the scan line (130) along the plurality of predetermined processing paths (160), which are arranged such that the plurality of resulting pockets (170) are at least partially connected with each other, and / or wherein the electronic circuit (105) is further configured to control the ophthalmological device (100) to vary one of the geometrical parameters of the scan line (130) such that the plurality of resulting pockets (170) are at least partially connected with each other, thereby forming a connected pocket (171).

9. The ophthalmological device (100) according to claim 8, wherein the electronic circuit (105) is further configured to control the ophthalmological device (100), in particular the second scanner device (104b), to move the scan line (130) along the plurality of predetermined processing paths (160), which are arranged around the center of the cornea (202), such that the connected pocket (171) has at least partially a circular shape.

10. The ophthalmological device (100) according to one of the claims 8 or 9, wherein the electronic circuit (105) is further configured to control the ophthalmological device (100) to move the scan line (130) along the plurality of predetermined processing paths (160), which are arranged around the center of the cornea (202) and wherein the processing paths (160) end prior of reaching the center area of the cornea (210) such that the resulting connected pocket (171) has at least partially a circular ring shape.

11. The ophthalmological device (100) according to any one of the preceding claims, wherein the electronic circuit (105) is further configured to control the scanner system (104) and / or the focusing optics (103) to vary a depth position of the focal spot (S) within the cornea (202) along the predetermined processing path (160).

12. The ophthalmological device (100) according to any one of the preceding claims, wherein the electronic circuit (105) is further configured to control the ophthalmological device (100) to move the scan line (130) along the at least one processing path (160), which is arranged in the cornea (202) having a predetermined, preferably constant, distance from the outer surface (209) of the cornea (202), thereby following the outer contour of the cornea (202) towards the center of the cornea (202).

13. The ophthalmological device (100) according to any one of the preceding claims, wherein the ophthalmological device (100) further comprises a patient interface (120), the patient interface (120) comprising a contact body (121) and one or more suction elements configured to fix the contact body to the cornea (202) for contacting the cornea (202) in a contacting zone where the contact body is in contact, preferably in applanating contact, with the outer surface (209) of the cornea (202), and wherein the electronic circuit (105) is further configured to control the scanner system (104) to move the scan line (130) along the predetermined processing path (160) at least partially inside the contact zone.

14. The ophthalmological device (100) according to any one of the preceding claims, wherein the electronic circuit (105) is further configured to control the scanner system (104) to move the scan line (130) along at least one predetermined access path (160), which extends from the outer surface (209) of the cornea (202) into the cornea (202) thereby connecting the surrounding with the at least one pocket (170) enabling access to the pocket (170).

15. A computer program product comprising a non-transitory computer-readable medium having stored thereon computer program code for controlling a processor (106) of an ophthalmological device (100), which comprises a laser source (102) configured to generate a pulsed laser beam (T), a focusing optics (103) configured to make the pulsed laser beam (T) converge onto a focal spot (S) in the cornea (202), a scanner system (104) configured to deflect the pulsed laser beam (T) to target locations in the cornea (202), whereby the computer program code is configured to control the processor (106) such that the processor (106):
directs a fist scanner device (104a) of the scanner system (104) to move the focal spot (S) with a first scanning speed to target locations along a line, thereby forming a scan line (130), and
directs a second scanner device (104b) of the scanner system (104) to move the scan line (130) with a second scanning speed, comparatively slower than the first scanning speed, along a predetermined processing path (160), which extends inside the cornea (202) from a peripheral portion of the cornea (202) towards a center of the cornea (202) or vice versa, for treating the cornea (202), in particular for cutting a pocket (170) inside the cornea (202), and
and directs the ophthalmological device (100) to vary at least one of: a geometrical parameter of the scan line (130) or a physical parameter of the scan line (130), along the predetermined processing path (160) through the cornea (202) and
directs the ophthalmological device (100) to vary the geometrical parameter of the scan line (130), in particular a length (132) of the scan line and/or a scan line orientation angle (β), along the predetermined processing path (160) such that the length (132) of the scan line (130) shortens in the direction towards the center of the cornea (202), and/or extends in the direction away from the center of the cornea (202), thereby forming at least partially a triangular shaped or a trapezoidal shaped pocket (170), and/or
directs the scanner system (104) to move the scan line (130) along a plurality of predetermined processing paths (160), which are arranged at least partially next to each other, thereby forming a plurality of resulting pockets (170) in the cornea (202).

## Patentansprüche

1. Ophthalmologische Vorrichtung (100) zur chirurgischen Behandlung einer Hornhaut (202) eines Auges (201) eines Patienten (200), wobei die ophthalmologische Vorrichtung (100) umfasst:
a. eine Laserquelle (102), die eingerichtet ist einen gepulsten Laserstrahl (T) zu erzeugen;
b. eine Fokussierungsoptik (103), die eingerichtet ist den gepulsten Laserstrahl (T) auf einen Brennpunkt (S) in der Hornhaut (202) zu konvergieren;
c. ein Scannersystem (104), das eingerichtet ist den gepulsten Laserstrahl (T) auf Zielstellen in der Hornhaut (202) abzulenken; und
d. eine elektronische Schaltung (105), die eingerichtet ist:
i. eine erste Scanvorrichtung (104a) des Scannersystems (104) zu steuern, um den Brennpunkt (S) mit einer ersten Scangeschwindigkeit entlang einer Linie zu bewegen, wodurch eine Scanlinie (130) gebildet wird,
ii. eine zweite Scanvorrichtung (104b) des Scannersystems (104) zu steuern, um die Scanlinie (130) mit einer zweiten Scangeschwindigkeit, die vergleichsweise langsamer als die erste Scangeschwindigkeit ist, entlang eines vorbestimmten Bearbeitungspfades (160) zu bewegen, der innerhalb der Hornhaut (202) von einem peripheren Teil der Hornhaut (202) zu einer Mitte der Hornhaut (202) oder umgekehrt verläuft, um die Hornhaut (202) zu behandeln, insbesondere um eine Tasche (170) innerhalb der Hornhaut (202) zu schneiden, und
iii. die ophthalmologische Vorrichtung (100) zu steuern, um mindestens einen der folgenden Parameter zu variieren: einen geometrischen Parameter der Scanlinie (130) oder einen physikalischen Parameter der Scanlinie (130), entlang des vorbestimmten Bearbeitungspfads (160) durch die Hornhaut (202), und
iv. die ophthalmologische Vorrichtung (100) zu steuern, um den geometrischen Parameter der Scanlinie (130) entlang des vorbestimmten Bearbeitungspfades (160) derart zu variieren, dass sich eine Länge (132) der Scanlinie (130) in der Richtung zur Mitte der Hornhaut (202) hin verkürzt und/oder in der Richtung von der Mitte der Hornhaut (202) weg erstreckt, wodurch zumindest teilweise eine dreieckig oder trapezförmig geformte Tasche (170) gebildet wird, und/oder
v. das Scannersystem (104) zu steuern, um die Scanlinie (130) entlang einer Mehrzahl von vorbestimmten Bearbeitungspfaden (160) zu bewegen, die zumindest teilweise nebeneinander angeordnet sind, wodurch eine Mehrzahl von resultierenden Taschen (170) in der Hornhaut (202) gebildet wird.

2. Die ophthalmologische Vorrichtung (100) gemäß Anspruch 1, wobei die geometrischen Parameter der Scanlinie (130) mindestens eines der folgenden umfassen: die Länge (132) der Scanlinie (130), einen Orientierungswinkel (β) der Scanlinie (130) in Bezug auf den vorbestimmten Bearbeitungspfad (160), eine Krümmung (136) der Scanlinie (130), eine Position (138) der Scanlinie (130) in Bezug auf den vorbestimmten Bearbeitungspfad (160) oder eine Tiefe (140) des Brennpunkts (S) in der Hornhaut (202).

3. Die ophthalmologische Vorrichtung (100) gemäß einem der vorhergehenden Ansprüche, wobei die physikalischen Parameter der Scanlinie (130) mindestens einen der folgenden Parameter umfassen: eine Impulsenergiemenge (142) pro Impuls des gepulsten Laserstrahls (T) oder eine Impulsintervallzeit zwischen Impulsen des gepulsten Laserstrahls (T).

4. Die ophthalmologische Vorrichtung (100) gemäß einem der vorhergehenden Ansprüche, wobei die elektronische Schaltung (105) dazu eingerichtet ist die erste Scanvorrichtung (104a) oder eine spezifische Vorrichtung zur Bestimmung der geometrischen Parameter (123) der ophthalmologischen Vorrichtung (100) zu steuern, um mindestens einen der geometrischen Parameter der Scanlinie (130) zu variieren, insbesondere um die Länge (132) der Scanlinie (130) entlang des vorbestimmten Bearbeitungspfades (160) durch die Hornhaut (202) zu variieren.

5. Die ophthalmologische Vorrichtung (100) gemäß einem der vorhergehenden Ansprüche, wobei die elektronische Schaltung (105) ferner dazu eingerichtet ist die zweite Scanvorrichtung (104b) zu steuern, um die Scanlinie (130) entlang des vorbestimmten Bearbeitungspfades (160) zu bewegen, der von einem ersten peripheren Punkt der Hornhaut (220) zu einem diametral gegenüberliegend angeordneten zweiten peripheren Punkt der Hornhaut (222) verläuft, wodurch die Mitte, insbesondere der Mittelpunkt oder der zentrale Bereich (210), der Hornhaut (210) gekreuzt wird.

6. Die ophthalmologische Vorrichtung (100) gemäß einem der vorhergehenden Ansprüche, wobei die elektronische Schaltung (105) ferner dazu eingerichtet ist das Scannersystem (104) derart zu steuern, dass der Scanlinienorientierungswinkel (β) in Bezug auf den vorbestimmten Bearbeitungspfad (160) während der Bewegung der Scanlinie (130) entlang des vorbestimmten Bearbeitungspfades (160) konstant gehalten wird, insbesondere so, dass der Scanlinienorientierungswinkel (β) in Bezug auf den vorbestimmten Bearbeitungspfad (160) bei 90° bleibt.

7. Die ophthalmologische Vorrichtung (100) gemäß einem der vorhergehenden Ansprüche, wobei die elektronische Schaltung (105) ferner dazu eingerichtet ist die ophthalmologische Vorrichtung (100), insbesondere die zweite Scanvorrichtung (104b), zu steuern, um die Scanlinie (130) entlang der Mehrzahl von vorbestimmten Bearbeitungspfaden (160) zu bewegen, die variierende Längen (160) aufweisen und/oder eine unterschiedliche Ausrichtung in Bezug auf das Auge (201) aufweisen, so dass die Mehrzahl der resultierenden Taschen (170) unterschiedliche geometrische Ausdehnungen aufweisen.

8. Die ophthalmologische Vorrichtung (100) gemäß einem der vorhergehenden Ansprüche, wobei die elektronische Schaltung (105) ferner dazu eingerichtet ist die ophthalmologischen Vorrichtung (100), insbesondere der zweiten Scanvorrichtung (104b), zu steuern, um die Scanlinie (130) entlang der Mehrzahl von vorbestimmten Bearbeitungspfaden (160) zu bewegen, die derart angeordnet sind, dass die Mehrzahl der resultierenden Taschen (170) zumindest teilweise miteinander verbunden sind, und/oder wobei die elektronische Schaltung (105) ferner dazu eingerichtet ist die ophthalmologische Vorrichtung (100) zu steuern, um einen der geometrischen Parameter der Scanlinie (130) derart zu variieren, dass die Mehrzahl der resultierenden Taschen (170) zumindest teilweise miteinander verbunden sind, wodurch eine verbundene Tasche (171) gebildet wird.

9. Die ophthalmologische Vorrichtung (100) gemäß Anspruch 8, wobei die elektronische Schaltung (105) ferner dazu eingerichtet ist die ophthalmologische Vorrichtung (100), insbesondere der zweiten Scanvorrichtung (104b), zu steuern, um die Scanlinie (130) entlang der Mehrzahl von vorbestimmten Bearbeitungspfaden (160) zu bewegen, die um die Mitte der Hornhaut (202) herum angeordnet sind, so dass die verbundene Tasche (171) zumindest teilweise eine kreisförmige Form aufweist.

10. Die ophthalmologische Vorrichtung (100) gemäß einem der Ansprüche 8 oder 9, wobei die elektronische Schaltung (105) ferner dazu eingerichtet ist die ophthalmologische Vorrichtung (100) zu steuern, um die Scanlinie (130) entlang der Mehrzahl von vorbestimmten Bearbeitungspfaden (160) zu bewegen, die um die Mitte der Hornhaut (202) angeordnet sind, und wobei die Bearbeitungspfade (160) vor dem Erreichen des zentralen Bereichs der Hornhaut (210) enden, so dass die resultierende verbundene Tasche (171) zumindest teilweise eine kreisförmige Ringform aufweist.

11. Die ophthalmologische Vorrichtung (100) gemäß einem der vorhergehenden Ansprüche, wobei die elektronische Schaltung (105) ferner dazu eingerichtet ist das Scannersystem (104) und/oder die Fokussieroptik (103) zu steuern, um eine Tiefenposition des Brennpunktes (S) innerhalb der Hornhaut (202) entlang des vorbestimmten Bearbeitungspfades (160) zu variieren.

12. Die ophthalmologische Vorrichtung (100) gemäß einem der vorhergehenden Ansprüche, wobei die elektronische Schaltung (105) ferner dazu eingerichtet ist die ophthalmologische Vorrichtung (100) zu steuern, um die Scanlinie (130) entlang des mindestens einen Bearbeitungspfades (160) zu bewegen, der in der Hornhaut (202) angeordnet ist und einen vorbestimmten, vorzugsweise konstanten Abstand von der äußeren Oberfläche (209) der Hornhaut (202) hat, wodurch er der äußeren Kontur der Hornhaut (202) in Richtung der Mitte der Hornhaut (202) folgt.

13. Die ophthalmologische Vorrichtung (100) gemäß einem der vorhergehenden Ansprüche, wobei die ophthalmologische Vorrichtung (100) ferner eine Patientenschnittstelle (120) umfasst, wobei die Patientenschnittstelle (120) einen Kontaktkörper (121) und ein oder mehrere Saugelemente umfasst, die dazu eingerichtet sind den Kontaktkörper an der Hornhaut (202) zu fixieren, um die Hornhaut (202) in einer Kontaktzone zu kontaktieren, in der der Kontaktkörper in Kontakt, vorzugsweise in ebnenden Kontakt, mit der äusseren Oberfläche (209) der Hornhaut (202) ist, und wobei die elektronische Schaltung (105) ferner zur Kontrolle des Scannersystems (104) eingerichtet ist, um die Scanlinie (130) zumindest teilweise innerhalb der Kontaktzone entlang des vorbestimmten Bearbeitungspfades (160) zu bewegen.

14. Die ophthalmologische Vorrichtung (100) gemäß einem der vorhergehenden Ansprüche, wobei die elektronische Schaltung (105) ferner dazu eingerichtet ist das Scannersystem (104) zu steuern, um die Scanlinie (130) entlang mindestens eines vorbestimmten Zugangspfades (160) zu bewegen, der von der äußeren Oberfläche (209) der Hornhaut (202) in die Hornhaut (202) verläuft, wodurch die Umgebung mit der mindestens einen Tasche (170) verbunden wird, was den Zugang zu der Tasche (170) ermöglicht.

15. Computerprogrammprodukt, umfassend ein nichtflüchtiges computerlesbares Medium, auf dem ein Computerprogrammcode zur Kontrolle eines Prozessors (106) einer ophthalmologischen Vorrichtung (100) gespeichert ist, die eine Laserquelle (102) umfasst, die eingerichtet ist, einen gepulsten Laserstrahl (T) zu erzeugen; eine Fokussierungsoptik (103), die eingerichtet ist, den gepulsten Laserstrahl (T) auf einen Brennpunkt (S) in der Hornhaut (202) zu konvergieren, ein Scannersystem (104), das eingerichtet ist, den gepulsten Laserstrahl (T) auf Zielstellen in der Hornhaut (202) abzulenken, wobei der Computerprogrammcode eingerichtet ist, den Prozessor (106) so zu steuern, dass der Prozessor (106) eine erste Scanvorrichtung (104a) des Scannersystems (104) anweist, den Brennpunkt (S) mit einer ersten Scangeschwindigkeit zu Zielstellen entlang einer Linie zu bewegen, wodurch eine Scanlinie (130) gebildet wird, und
eine zweite Scanvorrichtung (104b) des Scannersystems (104) anweist, die Scanlinie (130) mit einer zweiten Scangeschwindigkeit, die vergleichsweise langsamer als die erste Scangeschwindigkeit ist, entlang eines vorbestimmten Bearbeitungspfades (160) zu bewegen, der innerhalb der Hornhaut (202) von einem peripheren Teil der Hornhaut (202) zu einer Mitte der Hornhaut (202) oder umgekehrt verläuft, um die Hornhaut (202) zu behandeln, insbesondere um eine Tasche (170) innerhalb der Hornhaut (202) zu schneiden, und
und die ophthalmologische Vorrichtung (100) anweist, mindestens einen der folgenden Parameter zu variieren: einen geometrischen Parameter der Scanlinie (130) oder einen physikalischen Parameter der Scanlinie (130), entlang des vorbestimmten Bearbeitungspfads (160) durch die Hornhaut (202) und
die ophthalmologische Vorrichtung (100) anweist, den geometrischen Parameter der Scanlinie (130), insbesondere eine Länge (132) der Scanlinie und/oder einen Scanlinienorientierungswinkel (β), entlang des vorbestimmten Bearbeitungspfads (160) zu variieren, so dass sich die Länge (132) der Scanlinie (130) in der Richtung zur Mitte der Hornhaut (202) hin verkürzt und/oder in der Richtung von der Mitte der Hornhaut (202) weg erstreckt, wodurch zumindest teilweise eine dreieckig oder trapezförmig geformte Tasche (170) gebildet wird, und/oder
das Scannersystem (104) anweist, die Scanlinie (130) entlang einer Mehrzahl von vorbestimmten Bearbeitungspfaden (160) zu bewegen, die zumindest teilweise nebeneinander angeordnet sind, wodurch eine Mehrzahl von resultierenden Taschen (170) in der Hornhaut (202) gebildet wird.

## Revendications

1. Dispositif ophtalmologique (100) pour le traitement chirurgical d'une cornée (202) d'un œil (201) d'un patient (200), le dispositif ophtalmologique (100) comprenant :
a. une source laser (102) configurée pour générer un faisceau laser pulsé (T) ;
b. une optique de focalisation (103) configurée pour faire converger le faisceau laser pulsé (T) vers un point focal (S) dans la cornée (202) ;
c. un système de balayage (104) configuré pour dévier le faisceau laser pulsé (T) vers des positions cibles dans la cornée (202) ; et
d. un circuit électronique (105) configuré pour
i. commander un premier dispositif de balayage (104a) du système de balayage (104) pour déplacer le point focal (S) avec une première vitesse de balayage le long d'une ligne, formant ainsi une ligne de balayage (130),
ii. commander un deuxième dispositif de balayage (104b) du système de balayage (104) pour déplacer la ligne de balayage (130) à une deuxième vitesse de balayage, comparativement plus lente que la première vitesse de balayage, le long d'un chemin de traitement prédéterminé (160), qui s'étend à l'intérieur de la cornée (202) d'une partie périphérique de la cornée (202) vers un centre de la cornée (202) ou vice versa, pour traiter la cornée (202), en particulier pour découper une poche (170) à l'intérieur de la cornée (202), et
iii. commander le dispositif ophtalmologique (100) pour faire varier au moins l'un des paramètres suivants : un paramètre géométrique de la ligne de balayage (130) ou un paramètre physique de la ligne de balayage (130), le long du chemin de traitement prédéterminé (160) à travers la cornée (202), et
iv. commander le dispositif ophtalmologique (100) pour faire varier le paramètre géométrique de la ligne de balayage (130) le long du chemin de traitement prédéterminé (160) de sorte qu'une longueur (132) de la ligne de balayage (130) se raccourcisse en direction du centre de la cornée (202), et/ou s'étende en direction opposée au centre de la cornée (202), formant ainsi au moins partiellement une poche de forme triangulaire ou trapézoïdale (170), et/ou
v. commander le système de balayage (104) pour déplacer la ligne de balayage (130) le long d'une pluralité de chemins de traitement prédéterminés (160), qui sont disposés au moins partiellement les uns à côté des autres, formant ainsi une pluralité de poches résultantes (170) dans la cornée (202).

2. Dispositif ophtalmologique (100) selon la revendication 1, dans lequel les paramètres géométriques de la ligne de balayage (130) comprennent au moins l'un parmi : la longueur (132) de la ligne de balayage (130), un angle d'orientation (β) de la ligne de balayage (130) par rapport au chemin de traitement prédéterminé (160), une courbure (136) de la ligne de balayage (130), une position (138) de la ligne de balayage (130) par rapport au chemin de traitement prédéterminé (160) ou une profondeur (140) du point focal (S) dans la cornée (202).

3. Dispositif ophtalmologique (100) selon l'une quelconque des revendications précédentes, dans lequel les paramètres physiques de la ligne de balayage (130) comprennent au moins l'un parmi : une quantité d'énergie d'impulsion (142) par impulsion du faisceau laser pulsé (T) ou un temps d'intervalle d'impulsion entre les impulsions du faisceau laser pulsé (T).

4. Dispositif ophtalmologique (100) selon l'une quelconque des revendications précédentes, dans lequel le circuit électronique (105) est configuré pour commander le premier dispositif de balayage (104a) ou un dispositif spécifique de détermination de paramètres géométriques (123) du dispositif ophtalmologique (100) pour faire varier au moins un des paramètres géométriques de la ligne de balayage (130), en particulier pour faire varier la longueur (132) de la ligne de balayage (130) le long du chemin de traitement prédéterminé (160) à travers la cornée (202).

5. Dispositif ophtalmologique (100) selon l'une quelconque des revendications précédentes, dans lequel le circuit électronique (105) est en outre configuré pour commander le deuxième dispositif de balayage (104b) pour déplacer la ligne de balayage (130) le long du chemin de traitement prédéterminé (160), qui s'étend d'un premier point périphérique de la cornée (220) vers un deuxième point périphérique de la cornée (222) agencé de manière diamétralement opposée, traversant ainsi le centre, en particulier le point central ou la zone centrale (210), de la cornée (210).

6. Dispositif ophtalmologique (100) selon l'une quelconque des revendications précédentes, dans lequel le circuit électronique (105) est en outre configuré pour commander le système de balayage (104) de sorte que l'angle d'orientation de la ligne de balayage (β) par rapport au chemin de traitement prédéterminé (160) soit maintenu constant pendant le déplacement de la ligne de balayage (130) le long du chemin de traitement prédéterminé (160), en particulier l'angle d'orientation de la ligne de balayage (β) reste à 90° par rapport au chemin de traitement prédéterminé (160).

7. Dispositif ophtalmologique (100) selon l'une quelconque des revendications précédentes, dans lequel le circuit électronique (105) est en outre configuré pour commander le dispositif ophtalmologique (100), en particulier le deuxième dispositif de balayage (104b), pour déplacer la ligne de balayage (130) le long de la pluralité de chemins de traitement prédéterminés (160) ayant des longueurs variables (160), et / ou ayant une orientation différente par rapport à l'œil (201) de sorte que la pluralité de poches résultantes (170) ont des extensions géométriques différentes.

8. Dispositif ophtalmologique (100) selon l'une quelconque des revendications précédentes, dans lequel le circuit électronique (105) est en outre configuré pour commander le dispositif ophtalmologique (100), en particulier le deuxième dispositif de balayage (104b), pour déplacer la ligne de balayage (130) le long de la pluralité de chemins de traitement prédéterminés (160), qui sont disposés de manière à ce que la pluralité de poches résultantes (170) soient au moins partiellement connectées les unes aux autres, et / ou dans lequel le circuit électronique (105) est en outre configuré pour commander le dispositif ophtalmologique (100) afin de faire varier l'un des paramètres géométriques de la ligne de balayage (130) de manière à ce que la pluralité de poches résultantes (170) soient au moins partiellement connectées les unes aux autres, formant ainsi une poche connectée (171).

9. Dispositif ophtalmologique (100) selon la revendication 8, dans lequel le circuit électronique (105) est en outre configuré pour commander le dispositif ophtalmologique (100), en particulier le deuxième dispositif de balayage (104b), pour déplacer la ligne de balayage (130) le long de la pluralité de chemins de traitement prédéterminés (160), qui sont disposées autour du centre de la cornée (202), de sorte que la poche connectée (171) présente au moins partiellement une forme circulaire.

10. Le dispositif ophtalmologique (100) selon l'une des revendications 8 ou 9, dans lequel le circuit électronique (105) est en outre configuré pour commander le dispositif ophtalmologique (100) afin de déplacer la ligne de balayage (130) le long de la pluralité de chemins de traitement prédéterminés (160), qui sont disposés autour du centre de la cornée (202) et dans lequel les chemins de traitement (160) se terminent avant d'atteindre la zone centrale de la cornée (210) de sorte que la poche connectée résultante (171) a au moins partiellement une forme d'anneau circulaire.

11. Le dispositif ophtalmologique (100) selon l'une quelconque des revendications précédentes, dans lequel le circuit électronique (105) est en outre configuré pour commander le système de balayage (104) et / ou l'optique de focalisation (103) pour faire varier une position en profondeur du point focal (S) à l'intérieur de la cornée (202) le long du chemin de traitement prédéterminé (160).

12. Le dispositif ophtalmologique (100) selon l'une quelconque des revendications précédentes, dans lequel le circuit électronique (105) est en outre configuré pour commander le dispositif ophtalmologique (100) afin de déplacer la ligne de balayage (130) le long de l'au moins un chemin de traitement (160), qui est disposé dans la cornée (202) à une distance prédéterminée, de préférence constante, de la surface extérieure (209) de la cornée (202), suivant ainsi le contour extérieur de la cornée (202) vers le centre de la cornée (202).

13. Dispositif ophtalmologique (100) selon l'une quelconque des revendications précédentes, dans lequel le dispositif ophtalmologique (100) comprend en outre une interface patient (120), l'interface patient (120) comprenant un corps de contact (121) et un ou plusieurs éléments d'aspiration configurés pour fixer le corps de contact sur la cornée (202) pour entrer en contact avec la cornée (202) dans une zone de contact où le corps de contact est en contact, de préférence en contact applanissant, avec la surface extérieure (209) de la cornée (202), et dans laquelle le circuit électronique (105) est en outre configuré pour commander le système de balayage (104) afin de déplacer la ligne de balayage (130) le long du chemin de traitement prédéterminé (160) au moins partiellement à l'intérieur de la zone de contact.

14. Dispositif ophtalmologique (100) selon l'une quelconque des revendications précédentes, dans lequel le circuit électronique (105) est en outre configuré pour commander le système de balayage (104) pour déplacer la ligne de balayage (130) le long d'au moins un chemin d'accès prédéterminé (160), qui s'étend de la surface extérieure (209) de la cornée (202) à l'intérieur de la cornée (202) reliant ainsi l'environnement avec l'au moins une poche (170) permettant l'accès à la poche (170).

15. Produit programme d'ordinateur comprenant un support lisible par ordinateur non transitoire sur lequel est stocké un code programme d'ordinateur pour commander un processeur (106) d'un dispositif ophtalmologique (100), qui comprend une source laser (102) configurée pour générer un faisceau laser pulsé (T), une optique de focalisation (103) configurée pour faire converger le faisceau laser pulsé (T) vers un point focal (S) dans la cornée (202), un système de balayage (104) configuré pour dévier le faisceau laser pulsé (T) vers des positions cibles dans la cornée (202), par lequel le code de programme informatique est configuré pour commander le processeur (106) de telle sorte que le processeur (106) :
ordonne à un premier dispositif de balayage (104a) du système de balayage (104) de déplacer le point focal (S) avec une première vitesse de balayage vers des positions cibles le long d'une ligne, formant ainsi une ligne de balayage (130), et
ordonne à un deuxième dispositif de balayage (104b) du système de balayage (104) de déplacer la ligne de balayage (130) à une deuxième vitesse de balayage, comparativement plus lente que la première vitesse de balayage, le long d'un chemin de traitement prédéterminé (160), qui s'étend à l'intérieur de la cornée (202) d'une partie périphérique de la cornée (202) vers un centre de la cornée (202) ou vice versa, pour traiter la cornée (202), en particulier pour découper une poche (170) à l'intérieur de la cornée (202), et
et ordonne au dispositif ophtalmologique (100) de faire varier au moins l'un des paramètres suivants : un paramètre géométrique de la ligne de balayage (130) ou un paramètre physique de la ligne de balayage (130), le long du chemin de traitement prédéterminé (160) à travers la cornée (202), et
ordonne au dispositif ophtalmologique (100) de faire varier le paramètre géométrique de la ligne de balayage (130), en particulier une longueur (132) de la ligne de balayage et/ou un angle d'orientation de la ligne de balayage (β), le long du chemin de traitement prédéterminée (160) de sorte que la longueur (132) de la ligne de balayage (130) se raccourcisse en direction du centre de la cornée (202), et/ou s'étende en direction opposée au centre de la cornée (202), formant ainsi au moins partiellement une poche de forme triangulaire ou de forme trapézoïdale (170), et/ou
ordonne au système de balayage (104) de déplacer la ligne de balayage (130) le long d'une pluralité de chemins de traitement prédéterminés (160), qui sont disposés au moins partiellement les uns à côté des autres, formant ainsi une pluralité de poches résultantes (170) dans la cornée (202).
